(19) Europäisches Patentamt

European Patent Office

Office européen des brevets

(11) **EP 1 277 875 B1**

(12) **EUROPEAN PATENT SPECIFICATION**

(45) Date of publication and mention
of the grant of the patent:
**18.10.2006 Bulletin 2006/42**

(51) Int Cl.:
***D06M 13/342*** (2006.01)   ***D06M 15/263*** (2006.01)
***D06M 11/79*** (2006.01)   ***D06M 13/418*** (2006.01)

(21) Application number: **01926013.2**

(22) Date of filing: **27.04.2001**

(86) International application number:
**PCT/JP2001/003687**

(87) International publication number:
**WO 2001/083875 (08.11.2001 Gazette 2001/45)**

(54) **TEXTILE PRODUCT**

TEXTILPRODUKT

PRODUIT TEXTILE

(84) Designated Contracting States:
**DE FR IT**

(30) Priority: **28.04.2000 JP 2000013006**

(43) Date of publication of application:
**22.01.2003 Bulletin 2003/04**

(73) Proprietor: **Ajinomoto Co., Inc.
Tokyo 104-8315 (JP)**

(72) Inventors:
• **HIGUCHI, Ryouichi,
Amino Sc.Labs Ajinomoto Co.,Inc
Kawasaki-shi,
Kanagawa 210-8681 (JP)**
• **KITAMURA, Nobuyoshi,
Amino Science Labs
Kawasaki-shi,
Kanag awa 210-8681 (JP)**
• **HIROSE, Sadakazu,
R & D Department
Ayabe-shi,
Kyoto 623-0051 (JP)**
• **SUKEDA, Hideo,
R & D Department of Gunze Ltd
Ayabe-shi, Kyoto 623-0051 (JP)**
• **SUZUKI, Shiro,
R & D Department of Gunze Limited
Ayabe-shi,Kyoto 623-0051 (JP)**
• **ANNO, Katsuya,
Hosiery Division of Gunze Limited
Chuo-ku, Osaka-shi
Osaka 541-0055 (JP)**

(74) Representative: **Müller-Boré & Partner
Patentanwälte
Grafinger Strasse 2
81671 München (DE)**

(56) References cited:
EP-A- 1 118 705    JP-A- 9 157 152
JP-A- 11 093 075    JP-A- 2000 045 173
JP-A- 2000 199 178    JP-A- 2000 238 423
JP-A- 2000 265 380    US-A- 4 069 214
US-A- 5 306 444    US-A- 5 482 764

EP 1 277 875 B1

**Description**

The present invention relates to a textile product which is used to preserve skin moisture and a processing method for such a product, and in particular to a textile product having the effect of preserving normal skin by supplementing the moisture retention function of the stratum corneum and by enhancing metabolism.

Pyrrolidonecarboxylic acid (PCA) or amino acids such as arginine are natural moisturizing factors inherently found in the human body, and have been scrutinized for their skin care properties. Although the use of protein fibers and the provision of proteins to textile products are known (Japanese Unexamined Patent Publication H8-60547 and Japanese Examined Patent Publication H5-36534), for a long time, there had not been any textile products which have been endowed with skin care properties by processing a textile product using an amino acid such as arginine.

JP-A-2000 45173 describes textile products to which arginine has been applied. EP-A-1 118 705 describes a fiber treating agent having physiological skin protecting effects, comprised of an animal protein hydrolysate, chitosan lactate and one or more kinds of quaternary ammonium salts, and characterized in that said agent fixes natural skin moisturizing factor components onto clothes.

An object of the present invention is to provide a textile product which has the effect of preserving normal skin by supplementing the moisture retention function of the stratum corneum and by enhancing metabolism, while retaining the inherent properties of fiber materials, and in particular is to give a novel function to textile products which are close-fitting on the skin, such as underwear, stockings, socks, and gloves made of synthetic fibers which have poor moisture absorption, such as nylon and polyester.

Figure 1 shows the results of evaluation after wearing the tights of the present invention for a long period of time.
Figure 2 shows the results from test example 2.
Figure 3 is a photograph of a replica of a shin before wearing the tights of the invention.
Figure 4 is a photograph of a replica of a shin after wearing the tights of the invention for 14 days.
Figure 5 is a photograph of a replica of a heel before wearing the tights of the invention.
Figure 6 is a photograph of a replica of a heel after wearing the tights of the invention for 14 days.

The invention relates to the following matters 1 through 9.

1. A textile product comprising arginine in an amount of about 0.05 to 10 wt% relative to the fiber weight, and a cationic binder.
2. The textile product according to 1, further comprising pyrrolidonecarboxylic acid.
3. The textile product according to 1 or 2, comprising arginine in an amount of about 0.05 to 10 wt% relative to the fiber weight, and pyrrolidonecarboxylic acid in an amount of about 0.05 to 5 wt% relative to the fiber weight.
4. The textile product according to any one of 1 to 3, wherein the cationic binder is a cationic acrylic binder.
5. The textile product according to any one of 1 to 4, further comprising a silica dispersant.
6. A method for processing a textile product, characterized in that a textile product is pre-treated with a fiber pre-treatment agent comprising a cationic binder, and is then treated with a treatment solution comprising arginine and optionally, pyrrolidonecarboxylic acid.
7. The processing method according to 6, wherein the cationic binder is a cationic acrylic binder.
8. The processing method according to 6 or 7, wherein the fiber pre-treatment agent further contains a silica dispersant.
9. Use of the textile product according to anyone of 1 to 5 for skin care.

Examples of fiber materials include natural fibers such as cotton, flax, silk, and wool, and synthetic fibers such as nylon, rayon, polyester, cupra, acetate, and acrylic.

Examples of textile products include fibrils, yarn, pile, flocked material, weaves, knits, non-woven fabric, and cotton-like materials, and more specifically clothing such as underwear, athletic supports, socks, stockings, tights, and gloves.

The textile products in the present invention are pre-treated with a fiber pre-treatment agent to enhance the washability of the arginine, which results in textile products comprising arginine and a binder, and textile products comprising arginine, a binder, and a silica dispersant.

Examples of fiber pre-treatment agents include acrylic-, urethane-, polyester-, or epoxy-based binders, silica dispersants, and the like. The binder is a cationic binder (positively charged).

The textile product pre-treatment and the arginine and pyrrolidonecarboxylic acid treatment can involve the use of means such as dipping, coating, or spraying, but the textile is preferably treated by being dipped.

The textile product in the present invention should be pre-treated with a cationic acrylic binder and a silica dispersant, and should then be treated with arginine and, if needed, pyrrolidonecarboxylic acid. The pre-treatment can be managed, for example, by dipping a textile product for 5 to 30 minutes in a 40 to 80°C solution comprising a cationic acrylic binder and a silica dispersant, and then centrifugally drying the textile. The concentration of the cationic acrylic binder in the pre-treatment solution is between about 0.5 and 5 wt%, and the concentration of the silica dispersant is between about 0.5 and 5 wt%.

An example of a cationic acrylic binder is LIGHT-EPOCH BX-71 (tradename, by Kyoeisha Chemical Co., Ltd.).

An example of a silica dispersant is CLA-110 (tradename, by Kyoeisha Chemical Co., Ltd.).

When pre-treatment is undertaken with a fiber pre-treatment agent, the pre-treatment agent is left over in an amount of about 0.05 to 10 wt%, and preferably about 0.3 to 5 wt% of the fiber.

When the pre-treatment is undertaken with a fiber pre-treatment agent comprising a cationic acrylic binder, the amount of cationic acrylic binder in the fiber after the pre-treatment is about 0.05 to 5 wt%, and preferably about 0.3 to 2 wt%.

When the pre-treatment is undertaken with a fiber pre-treatment agent comprising a cationic acrylic binder and a silica dispersant, the amount of cationic acrylic binder in the fiber after the pre-treatment is about 0.05 to 5 wt%, and preferably about 0.3 to 2 wt%, and the amount of silica dispersant is 0.01 to 5 wt%, and preferably 0.02 to 0.5 wt%.

Such pre-treatment can improve the washability when chemicals are subsequently applied.

The arginine can be provided preferably by dipping the pre-treated fiber product for 5 to 30 minutes in a 40 to 60 °C treatment solution comprising 5 to 20 (g/L) of arginine, and then centrifugally drying the textile. When pyrrolidonecarboxylic acid is provided along with the arginine, the textile is similarly treated using a treatment solution comprising 5 to 20 (g/L) of arginine and 5 to 20 (g/L) of pyrrolidonecarboxylic acid. The pH of the treatment solution is preferably adjusted to between about 6.0 and 7.0 in order to prevent loss of dye. The treatment agent comprising the arginine may also include 2 to 5 wt% of softener (such as WS-937, by Marue Yuka KK).

The arginine may be free arginine, a salt with an inorganic acid such as hydrochloric acid, sulfuric acid, nitric acid, or phosphoric acid, an organic acid salt such as a citrate, succinate, p-toluenesulfonate, or methanesulfonate, or a salt with a base such as sodium or potassium.

The pyrrolidonecarboxylic acid may be in the form of a free pyrrolidonecarboxylic acid, or a salt with a base such as sodium or potassium.

The arginine is provided in an amount of about 0.05 to 10 wt%, and preferably about 2 to 10 wt%, relative to the fiber weight.

When pyrrolidonecarboxylic acid is provided along with the arginine, the arginine is provided in an amount of about 0.05 to 10 wt%, and preferably about 2 to 10 wt%, relative to the fiber weight, and the pyrrolidonecarboxylic acid is provided in an amount of about 0.05 to 5 wt%, and preferably about 0.5 to 5 wt%, relative to the fiber weight.

The arginine retention of the textile product in the present invention after 10 washings should be at least 60%, preferably at least 75%, and even more preferably at least 90%. The arginine retention after 20 washings should be at least 50%, preferably at least 65%, and even more preferably at least 80%.

The textile product of the present invention has better skin care properties such as better skin moisture content.

The textile product of the present invention pre-treated with the fiber pre-treatment agent has good washability and longer-lasting skin care properties.

The present invention is illustrated in further detail in the following examples.

Example 1

(1) Skin Care Processing

37.792 g nylon tights fabric was dipped in a 40°C treatment solution (380 mL) containing a cationic acrylic binder (2% owf, tradename: LIGHT-EPOCH BX-71 (by Kyoeisha Chemical Co., Ltd.)), silica dispersant (2% owf, tradename: CLA-110 (by Kyoeisha Chemical Co., Ltd.)), and softener (2% owf, tradename: WS-937, by Marue Yuka KK), and was then centrifugally dried for 15 seconds, giving fabric with a 50 to 60% moisture content. The resulting fabric was dipped for 30 minutes in 40°C treatment solution (pH 6.0) containing arginine (Arg: 10 g/L) and sodium pyrrolidonecarboxylate (PCA-Na: 15 g/L), and was then centrifugally dried for 15 seconds, giving a skin care processed tights fabric with a 50 to 60% moisture content, which was dried to give tights fabric for the test below.

Example 2

37.692 g nylon tights fabric was dipped, without being pre-treated, for 30 minutes in 40°C treatment solution (pH 6.0) containing arginine (Arg: 10 g/L) and sodium pyrrolidonecarboxylate (15 g/L) under the same conditions as in Example 1, and was then centrifugally dried for 15 seconds, giving a skin care processed tights fabric with a 50 to 60% moisture content, which was dried to give tights fabric for the following test.

Test Example 1: Laundering Test

According to JIS 0217 103 method, 5.0 g of the fabrics obtained in Examples 1 and 2 were washed for 5 minutes in 150 mL of 40°C tepid water at a bath ratio of 1:30 using 0.67 g/L (standard amount) of the detergent Attack (tradename, by Kao Corp.), and the fabrics wee then rinsed. The total amount of washing solution was 1460 mL. The fabrics were washed 20 times, and the amounts of arginine and pyrrolidonecarboxylic acid that were lost were measured by liquid chromatography to determine the retention.
The adhering amounts which are given in Table 2 were determined in the following manner.

<Method for Measurement of Adhering Amount>

The amounts that adhered were determined by subtracting the amounts of arginine and sodium pyrrolidonecarboxylate present in the treatment solution left over after the dipping and the treatment solution which was wrung from the textile from the amounts of arginine and sodium pyrrolidonecarboxylate present in the treatment solution before the tights fabrics were dipped. The amounts of arginine and sodium pyrrolidonecarboxylate were determined by measurement using liquid chromatography.
The results are given in Table 1.

Table 1

|  | Example 1 | Example 2 |
|---|---|---|
| Pre-treatment | yes | no |
| Pre-processing weight (g) | 37.792 | 37.692 |
| Amount adhering (g) (adhesion rate %) | | |
| Arg | 1.316 (3.48) | 0.442 (1.17) |
| PCA-Na | 0.363 (0.96) | 0.092 (0.24) |
| Retention (%) after 10 washings | | |
| Arg | 90.729 | 67.421 |
| PCA-Na | 72.452 | 0 |
| Retention (%) after 20 washings | | |
| Arg | 84.1 | - |
| PCA-Na | 60.3 | - |

Test Example 2: Skin Moisture Measuring Test

Three subjects washed their lower arms (3 cm $\times$ 3 cm) with 2 mL of sodium laurate 10% solution and dried them for 5 minutes, a washing step that was repeated 3 times to produce a model of dry skin, the fabric obtained in Examples 1 and 2 and unprocessed fabric were then applied, and the skin moisture content was measured with a SKICON-200 (tradename: by IBS) after 30 minutes and 1 hour. The measuring conditions involved a temperature of 20°C and 40% RH.
When the fabric of the present invention was applied, the skin moisture recovery rate was clearly better than when not applied. The results are given in Table 2.
The skin moisture recovery rate was measured in the following manner.

Measurement of Skin Moisture Recovery Rate

The skin moisture content X after the preparation of the model dry skin was first measured. The skin moisture content Y 30 minutes after the fabric obtained in Example 1 and unprocessed fabric had been applied was then measured, and the skin moisture recovery rate was determined using the following equation.

```
Skin moisture recovery rate (%) = ((Y-X)/X) × 100
```

Test Example 3: Long-Term Wearing Test

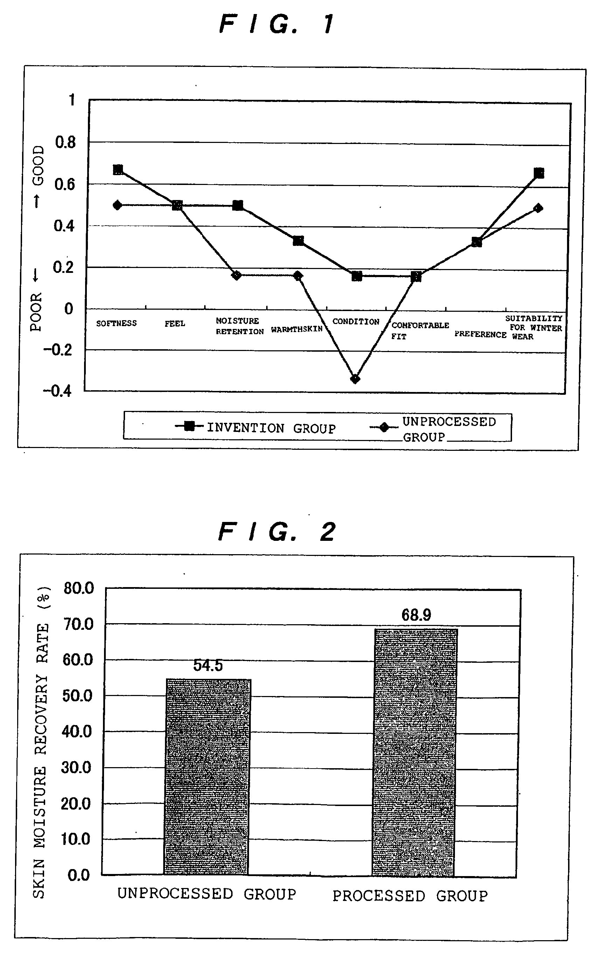

For two weeks, ten subjects wore tights made of the fabric obtained in Example 1 and unprocessed tights on alternating days for at least 8 hours a day to test 8 parameters consisting of softness, feel, moisture retention, warmth, skin condition, comfortable fit, preference, and suitability for winter wear. The skin moisture content of the heel after 2 weeks was determined using a SKICON-200 (tradename, by IBS). The measuring conditions involved a temperature of 20°C and 40% RH. The results are given in Table 2 and Figure 1. The tights were washed the day after being used and were worn again on the following day throughout the test period. The mean of the measured skin moisture content ($\mu$s) was obtained for the 10 subjects.

Table 2

| Tights | Measured skin moisture content ($\mu$s) |
|---|---|
| Example 1 | 39.17 |
| Unprocessed product | 32.56 |

Test Example 4: Long-Term Wearing Test With Dry Skin

Three subjects with dry skin from among the 10 subjects in Test Example 3 wore tights made of the fabric obtained in Example 1 (right leg: processed group) and unprocessed tights (left leg: unprocessed group) for 7 days each, at least 8 hours a day. The skin moisture content of the lower legs and heels was measured after 7 days using a SKICON-200. The results were given in Table 3. The skin moisture content ($\mu$s) given in Table 3 is the mean for the 3 subjects.

Table 3

| | Skin moisture content ($\mu$s) |
|---|---|
| Right lower leg | 2.33 |
| Right heel | 69.00 |
| Left lower leg | 0.11 |
| Left heel | 34.44 |

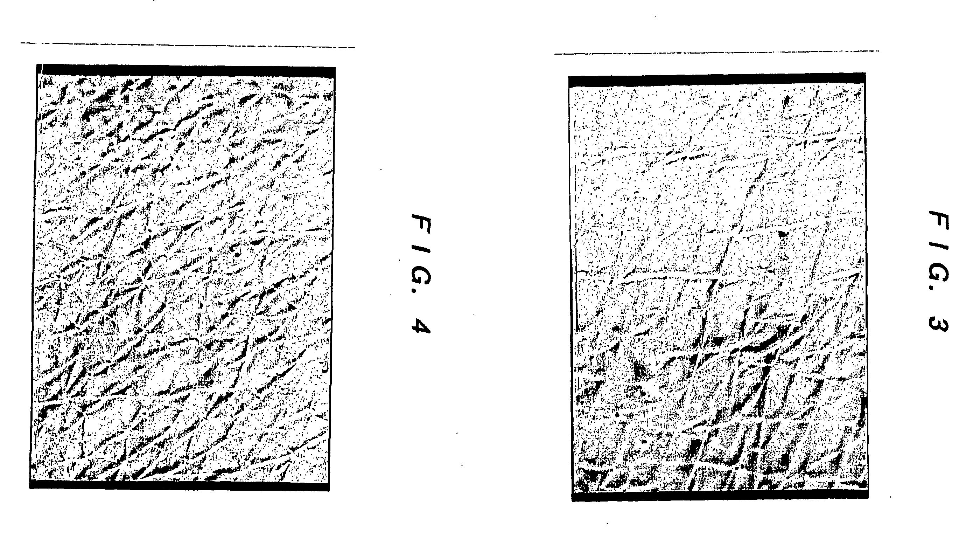

Figures 3 and 4 are photographs of a replica of a shin before and after the tights were worn.

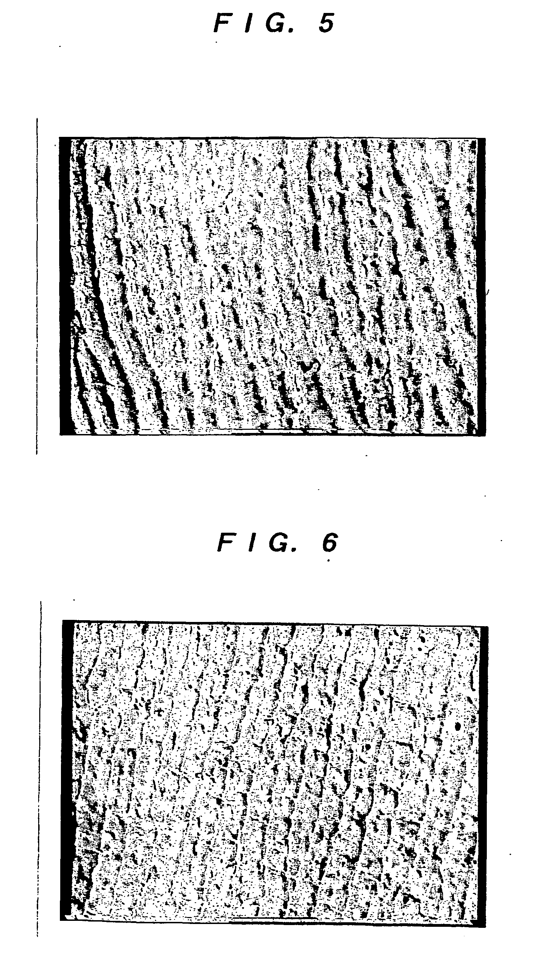

Figures 5 and 6 are photographs of a replica of a heel before and after the tights were worn.

**Claims**

1.  A textile product comprising arginine in an amount of about 0.05 to 10 wt% relative to the fiber weight, and a cationic binder.

2.  The textile product according to claim 1, further comprising pyrrolidonecarboxylic acid.

3.  The textile product according to claim 1 or 2, comprising arginine in an amount of about 0.05 to 10 wt% relative to the fiber weight, and pyrrolidonecarboxylic acid in an amount of about 0.05 to 5 wt% relative to the fiber weight.

4.  The textile product according to anyone of claims 1 to 3, wherein the cationic binder is a cationic acrylic binder.

5.  The textile product according to anyone of claims 1 to 4, further comprising a silica dispersant.

6.  A method for processing a textile product, **characterized in that** a textile product is pre-treated with a fiber pre-treatment agent comprising a cationic binder, and is then treated with a treatment solution comprising arginine and optionally, pyrrolidonecarboxylic acid.

7.  The processing method according to claim 6, wherein the cationic binder is a cationic acrylic binder.

8.  The processing method according to claim 6 or 7, wherein the fiber pre-treatment agent further contains a silica dispersant.

9. Use of the textile product according to anyone of claims 1 to 5 for skin care.

**Patentansprüche**

1. Textilprodukt, welches Arginin in einer Menge von etwa 0,05 bis 10 Gew.-%, bezogen auf das Fasergewicht, und ein kationisches Bindemittel umfasst.

2. Textilprodukt nach Anspruch 1, welches ferner Pyrrolidoncarbonsäure umfasst.

3. Textilprodukt nach Anspruch 1 oder 2, welches Arginin in einer Menge von etwa 0,05 bis 10 Gew.-%, bezogen auf das Fasergewicht, und Pyrrolidoncarbonsäure in einer Menge von etwa 0,05 bis 5 Gew.-%, bezogen auf das Fasergewicht, umfasst.

4. Textilprodukt nach einem der Ansprüche 1 bis 3, wobei das kationische Bindemittel ein kationisches Acrylbindemittel ist.

5. Textilprodukt nach einem der Ansprüche 1 bis 4, welches ferner ein Siliciumdioxid-Dispersionsmittel umfasst.

6. Verfahren zum Verarbeiten eines Textilproduktes, **dadurch gekennzeichnet, daß** ein Textilprodukt mit einem Faservorbehandlungsmittel, umfassend ein kationisches Bindemittel, vorbehandelt wird und anschließend mit einer Behandlungslösung, umfassend Arginin und gegebenenfalls Pyrrolidoncarbonsäure, behandelt wird.

7. Verarbeitungsverfahren nach Anspruch 6, wobei das kationische Bindemittel ein kationisches Acrylbindemittel ist.

8. Verarbeitungsverfahren nach Anspruch 6 oder 7, wobei das Faservorbehandlungsmittel ferner ein Siliciumdioxid-Dispersionsmittel enthält.

9. Verwendung des Textilproduktes nach einem der Ansprüche 1 bis 5 zur Hautpflege.

**Revendications**

1. Produit textile comprenant de l'arginine en une quantité d'environ 0,05 à 10 % en poids par rapport au poids de la fibre, et un liant cationique.

2. Produit textile selon la revendication 1, comprenant en outre l'acide pyrrolidonécarboxylique.

3. Produit textile selon la revendication 1 ou 2, comprenant de l'arginine en une quantité d'environ 0,05 à 10 % en poids par rapport au poids de la fibre, et l'acide pyrrolidonécarboxylique en une quantité d'environ 0,05 à 5 % en poids par rapport au poids de la fibre.

4. Produit textile selon l'une quelconque des revendications 1 à 3, dans lequel le liant cationique est un liant cationique acrylique.

5. Produit textile selon l'une quelconque des revendications 1 à 4, comprenant en outre un agent dispersant à base de silice.

6. Procédé de transformation d'un produit textile, **caractérisé en ce qu'**un produit textile est pré-traité avec un agent de pré-traitement de fibre comprenant un liant cationique, puis traité avec une solution de traitement comprenant de l'arginine et facultativement de l'acide pyrrolidonécarboxylique.

7. Procédé de transformation selon la revendication 6, dans lequel le liant cationique est un liant cationique acrylique.

8. Procédé de traitement selon la revendication 6 ou 7, dans lequel l'agent de pré-traitement de fibre contient en outre un agent de dispersion à base de silice.

9. Utilisation du produit textile selon l'une quelconque des revendications 1 à 5 pour le soin de la peau.

# F I G. 1

# F I G. 2

## F I G. 3

## F I G. 4

## F I G. 5

## F I G. 6